Europäisches Patentamt
European Patent Office
Office européen des brevets

(11) Veröffentlichungsnummer: **0 348 878 A2**

# EUROPÄISCHE PATENTANMELDUNG

(21) Anmeldenummer: 89111638.6

(22) Anmeldetag: 27.06.89

(51) Int. Cl.⁴: C07H 15/252, C07C 50/36, A61K 31/70

Patentansprüche für folgende Vertragsstaaten: ES + GR

(30) Priorität: 01.07.88 DE 3822220

(43) Veröffentlichungstag der Anmeldung: 03.01.90 Patentblatt 90/01

(84) Benannte Vertragsstaaten: AT BE CH DE ES FR GB GR IT LI LU NL SE

(71) Anmelder: BEHRINGWERKE Aktiengesellschaft
Postfach 1140
D-3550 Marburg 1(DE)

(72) Erfinder: Kolar, Cenek, Dr.
Deutschhausstrasse 20
D-3550 Marburg(DE)
Erfinder: Gerken, Manfred, Dr.
Wannkopfstrasse 12
D-3550 Marburg(DE)
Erfinder: Kraemer, Hans Peter, Dr. Dr.
Birkenweg 16
D-3550 Marburg(DE)
Erfinder: Hermentin, Peter, Dr.
Barfüssertor 30
D-3550 Marburg(DE)
Erfinder: Krohn, Karsten, Prof. Dr.
Brauerskamp 34
D-3300 Braunschweig(DE)
Erfinder: Linoh, Haryanto, Dr.
Helenenstrasse 22
D-3300 Braunschweig(DE)

(74) Vertreter: Klein, Otto, Dr. et al
Hoechst AG Zentrale Patentabteilung
Postfach 80 03 20
D-6230 Frankfurt am Main 80(DE)

(54) **Modifizierte Rhodomycine, Verfahren zu ihrer Herstellung und ihre Verwendung.**

(57) Es werden neue zytostatisch wirksame Anthracyclin-Derivate, die der nachfolgenden allgemeinen Formel I

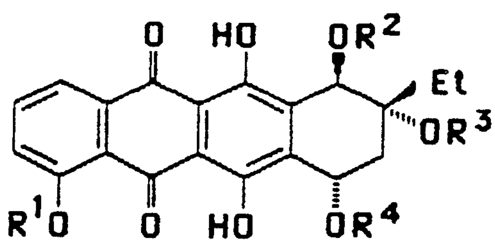

I

entsprechen, beschrieben, worin die Reste folgende Bedeutung haben:
$R^1$ ist eine $C_{1-4}$-Alkylgruppe;
$R^2$ ist ein Wasserstoffatom, eine Tri-$C_{1-4}$-alkylsilyl-Schutzgruppe, eine Acetyl-, Monohalogen-, Dihalogen- oder Trihalogen-acetylgruppe mit Halogen gleich Fluor oder Chlor, eine Benzoyl- oder p-Nitrobenzoylgruppe;
$R^3$ ist ein Wasserstoffatom;
$R^3$ und $R^4$ sind zusammen ein Phenylboronsäurerest PhB=;
$R^4$ ist ein Wasserstoffatom, eine Tri-$C_{1-4}$-alkylsilyl-Schutzgruppe oder ein Glycosyl-Rest der Formel II,

EP 0 348 878 A2

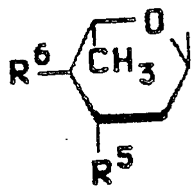

II

in der
$R^5$ eine Hydroxygruppe, eine Acetyloxygruppe, eine Aminogruppe, eine Trifluoracetamidogruppe, eine Di-$C_{1-4}$-alkylaminogruppe, eine 4-Morpholinylgruppe oder eine Cyanomethylaminogruppe und
$R^6$ ein Wasserstoffatom, eine Hydroxygruppe, eine Acetyloxy-, Trifluoracetyloxy- oder p-Nitrobenzoyloxy-Gruppe darstellen.

# Modifizierte Rhodomycine, Verfahren zu ihrer Herstellung und ihre Verwendung

Die vorliegende Erfindung bezieht sich auf neue zytostatisch wirksame Anthracyclin-Derivate, und sie betrifft speziell 4-0-Alkyl-7-0-glycosyl-rhodomycinone, Verfahren zu ihrer Herstellung sowie ihre Verwendung als Arzneimittel.

Die Substanzklasse der Anthracycline ist in der Fachliteratur eingehend beschrieben. Doxorubicin und sein 14-Desoxyanalogon, Daunorubicin, sind hier als die erfolgreichsten Vertreter dieser Substanzklasse genannt, die in der Klinik zur Behandlung einer großen Anzahl von festen Tumoren und Leukämien eingesetzt werden. Der Erfolg dieser speziellen Verbindungen ist jedoch typischerweise nicht bei allen Patienten gleich, und die Erfolgsrate ist bei einigen speziellen Tumorarten wie Dickdarmkrebs und Melanom geringer. Nebenwirkungen der Behandlung mit Doxorubicin und Daunorubicin sind u. a. eine Schädigung des Kreislaufsystems und dafür charakteristische Beschwerden.

Von der Doxorubicin-Daunorubicin-Gruppe ist bekannt, daß die 4-0-Methyl-Substitution in den Anthracyclinen für die antitumorale Wirkung und Tumorselektivität erforderlich ist. Die entsprechenden 4-Hydroxy-Derivate wie Carminomycin sind zytotoxischer und weniger selektiv.

Ausgehend von diesem Stand der Technik ist es die Aufgabe der vorliegenden Erfindung, von ß-Rhodomycinon als Ausgangsverbindung neue 4-0-Alkyl-rhodomycine zu schaffen, die sich durch ein neues Wirkungsspektrum und eine geringere Toxizität auszeichnen.

Überraschenderweise hat es sich bei der Alkylierung von 7,10-Bis-0-(trimethylsilyl)-ß-rhodomycinon gezeigt, daß von den in dem Molekül präsenten Hydroxygruppen (4-, 6-, 9- und 11-OH) bevorzugt die 4-OH-Gruppe alkyliert wird. Hiermit eröffnet sich ein Weg zur Herstellung und Anwendung von neuen Rhodomycin-Derivaten.

Aufbauend auf diesen Erkenntnissen hat es sich die vorliegende Erfindung zur Aufgabe gestellt, ausgehend von dem 4-0-Alkyl-ß-rhodomycinon 7-0-Glycosyl-Produkte herzustellen, ihr Wirkungsspektrum und ihre Anwendungsmöglichkeit als Tumor-therapeutisches Mittel zu untersuchen.

Gelöst wird diese Aufgabe mit neuen zytostatisch wirksamen Anthracyclin-Derivaten, die der nachfolgenden allgemeinen Formel I

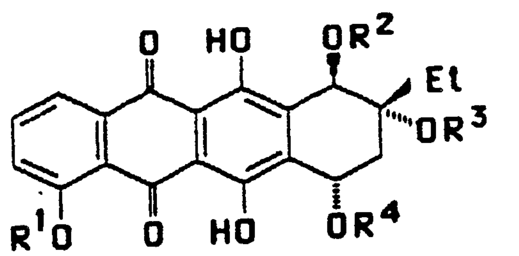

I

entsprechen, worin die Reste folgende Bedeutungen haben:

$R^1$ ist eine $C_{1-4}$-Alkylgruppe,

$R^2$ ist ein Wasserstoffatom, eine Tri-$C_{1-4}$-alkylsilyl-Schutzgruppe, eine Acetyl-, Monohalogen-, Dihalogen- oder Trihalogen-acetylgruppe mit Halogen gleich Fluor oder Chlor, eine Benzoyl- oder p-Nitrobenzoylgruppe,

$R^3$ ist ein Wasserstoffatom,

$R^3$ und $R^4$ sind zusammen ein Phenylboronsäurerest PhB=,

$R^4$ ist ein Wasserstoffatom, eine Tri-$C_{1-4}$-alkylsilyl-Schutzgruppe oder ein Glycosyl-Rest der Formel II,

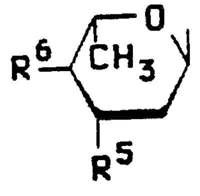

II

in der

$R^5$ eine Hydroxygruppe, eine Acetyloxygruppe, eine Aminogruppe, eine Trifluoracetamidogruppe, eine Di-

$C_{1-4}$-alkylaminogruppe, eine 4-Morpholinylgruppe oder eine Cyanomethylaminogruppe und $R^6$ ein Wasserstoffatom, eine Hydroxygruppe, eine Acetyloxy-, Trifluoracetyloxy- oder p-Nitrobenzoyloxy-Gruppe darstellen.

Bevorzugt sind Verbindungen der Formel I, worin
$R^1$ = $CH_3$-,
$R^2$ = $(Me)_3Si$- oder $F_3CCO$-,
$R^3$ = H,
$R^3$ und $R^4$ zusammen Ph-B=,
$R^4$ = H, $Me_3Si$- oder ein Glycosyl-Rest der Formel II mit
$R^5$ = OH, $CH_3COO$-, $NH_2$-, $F_3CCONH$-, $Me_2N$-, 4-Morpholinyl oder $NCCH_2NH$ und
$R^6$ = H, OH, $CH_3COO$-, $F_3CCOO$- oder $pNO_2$-PhCOO- sind.

Die Verbindungen der Formel I können gegebenenfalls als Ammoniumsalze vorliegen.

Das erfindungsgemäße Verfahren zur Herstellung einer der neuen Verbindungen der Formel I ist dadurch gekennzeichnet, daß man

a) ß-Rhodomycinon der Formel I, worin die Reste $R^1$, $R^2$, $R^3$ und $R^4$ ein Wasserstoffatom darstellen, mit Tri-$C_{1-4}$-alkylsilylchlorid in Gegenwart einer Base wie Pyridin und eines organischen Lösungsmittels wie Dichlormethan bei einer Temperatur zwischen -20° C und +25° C zum 7,10-Bis-0-tri-$C_{1-4}$-alkylsilyl-ß-rhodomycinon umsetzt, anschließend die entstandene Verbindung mit $C_{1-4}$-Alkylbromid oder -jodid in Gegenwart eines Alkalicarbonats zu einem 4-0-$C_{1-4}$-Alkyl-7,10-bis-0-(tri-$C_{1-4}$-alkylsilyl)-ß-rhodomycinon alkyliert und dann die Trialkylsilyl-Schutzgruppen säurehydrolytisch abspaltet, wobei eine 4-0-$C_{1-4}$-Alkyl-ß-rhodomycinon-Verbindung der Formel III entsteht,

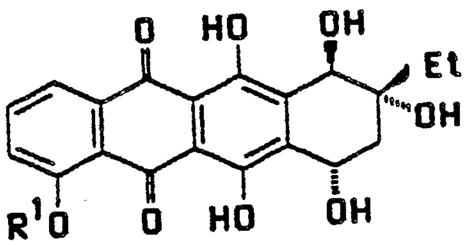

III,

worin $R^1$ eine $C_{1-4}$-Alkylgruppe darstellt,

b) die Verbindung der Formel III in an sich bekannter Weise mit Phenylboronsäure in Gegenwart einer Säure wie p-Toluolsulfonsäure oder Essigsäure und eines wasserfreien organischen Lösungsmittels wie Toluol selektiv an der 7- und 9-Hydroxygruppe verestert, anschließend die Verbindung mit einem Anhydrid oder Chlorid der Trifluoressig-, Essig-,Benzoe- oder p-Nitrobenzoe-Säure in Präsenz einer Base wie Triethylamin oder Pyridin an der 10-Hydroxygruppe acyliert und dann die Phenylboronyl-Schutzgruppe säurehydrolytisch oder mittels eines Diols wie 2-Methyl-pentan-2,4-diol abspaltet, wobei eine Verbindung der Formel IV entsteht,

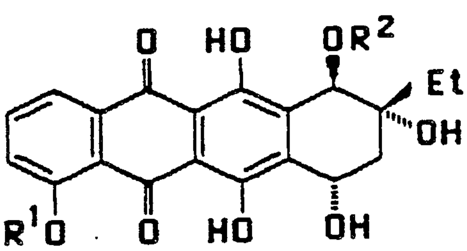

IV,

in der
$R^1$ eine $C_{1-4}$-Alkylgruppe und
$R^2$ eine Acylschutzgruppe, die sich von der Essig-, Trifluoressig-, Benzoe- oder p-Nitrobenzoesäure ableitet, bedeuten,

c) die Verbindung der Formel IV in an sich bekannter Weise mit einem funktionalisierten Desoxyzucker der Formel V oder VI,

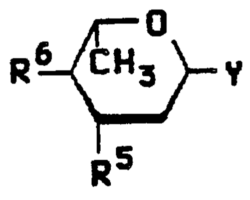

V

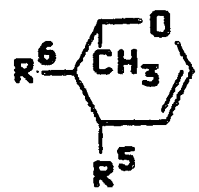

VI

worin die Reste
$R^5$ eine Acetyloxy- oder Trifluoracetamidogruppe,
$R^6$ ein Wasserstoffatom, eine Acetyloxy-, Trifluoracetyloxy- oder p-Nitrobenzoyloxygruppe und
Y eine Acetyloxy-, p-Nitrobenzoyloxygruppe oder ein Chlorid darstellen,
in Gegenwart eines Katalysators wie eines Trifluormethansulfonsäure-tri-$C_{1-4}$-alkylsilylesters oder des Silbersalzes der Trifluormethansulfonsäure zu einem 7-0-Glycosyl-ß-rhodomycinon-Derivat umsetzt und anschließend die Acylschutzgruppe alkalihydrolytisch abspaltet, wobei eine Verbindung der Formel VII entsteht,

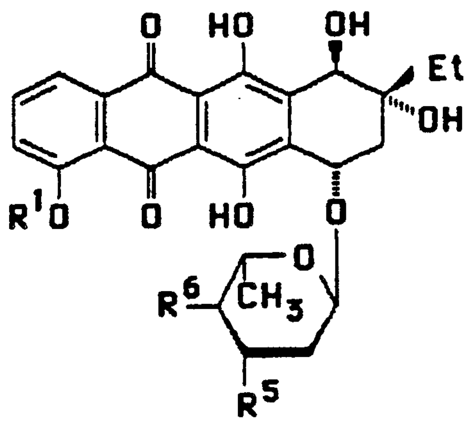

VII,

worin die Reste
$R^1$ eine $C_{1-4}$-Alkylgruppe,
$R^5$ eine Hydroxy- oder Aminogruppe und
$R^6$ ein Wasserstoffatom oder eine Hydroxygruppe darstellen,

d) eine einen Aminozucker enthaltende Verbindung der Formel VII in an sich bekannter Weise gegebenenfalls unter den Bedingungen der Alkylierung mit einem Halogenessigsäurenitril, oder der reduktiven Alkylierung mit $C_{1-4}$-Aldehyd oder Diglycolaldehyd in Gegenwart eines Alkalicyanoborhydrides zu einer weiteren Verbindung der Formel VII umsetzt, in der die Reste $R^1$ eine $C_{1-4}$-Alkylgruppe,
$R^5$ eine Cyanomethylaminogruppe, eine Di-$C_{1-4}$-alkylaminogruppe oder eine 4-Morpholinylgruppe und
$R^6$ ein Wasserstoffatom oder eine Hydroxygruppe darstellen.

Die Erfindung betrifft weiterhin pharmazeutische Zubereitungen, welche ein Anthracyclinglycosid der Formel I oder eines seiner pharmazeutisch annehmbaren Salze zusammen mit einem pharmazeutisch annehmbaren Verdünnungsmittel oder Träger enthalten. Diese Zubereitungen enthalten eine therapeutisch wirksame Menge des Anthracyclinglycosides oder seines Salzes. Die Erfindung betrifft weiterhin Verfahren für die Verwendung der Anthracyclinglycoside der Formel I oder ihrer Salze bei der Behandlung bestimmter Säugetiertumore durch Verabreichung einer therapeutisch wirksamen Menge an einen Patienten.

Die Bestimmung der zytostatischen Wirksamkeit der hier beschriebenen Verbindungen erfolgte in vitro an L1210-Leukämiezellen der Maus oder in vivo an L1210-Leukämie, B16-Melanoma und Lewis Lung Adenocarcinoma. Die akute Toxizität der Verbindungen wurde an NMRI-Mäusen ermittelt. Die Methoden sowie Ergebnisse dieser Untersuchungen sind in dem experimentellen Teil beschrieben.

## Beispiele

In den folgenden Beispielen wird die vorliegende Erfindung näher beschrieben, ohne sie hierbei einzuschränken.

Beispiel 1

Herstellung von 4-0-Alkyl-ß-rhodomycinonen

7,10-Bis-0-(trimethylsilyl)-ß-rhodomycinon (Verbindung 1)

Zu einer Lösung von 1000 mg (2,59 mmol) ß-Rhodomycinon in 10 ml Pyridin und 20 ml Dichlormethan wurden unter Rühren bei 0° C 10 ml (79,0 mmol) Trimethylchlorsilan zugetropft, und anschließend wurde die Reaktionsmischung 1 Stunde bei Raumtemperatur weitergerührt. Das in Dichlormethan aufgenommene Reaktionsgemisch wurde mit Eiswasser ausgewaschen, die organische Phase wurde über Natriumsulfat getrocknet und eingedampft. Den Rückstand trocknet man noch in Vakuum (0.1 Torr) bei 50° C. Das Rohprodukt wurde säulenchromatographisch an Kieselgel mit Dichlormethan als Eluiermittel gereinigt.
Ausbeute der Titelverbindung: 1130 mg, Schmelzpunkt: 185 - 186° C (Zersetzung). Die Titelverbindung wurde mittels $^1$H-NMR-, MS- sowie Elementaranalyse charakterisiert.
$^1$H-NMR (400 MHz,$CDCl_3$,delta(ppm)): 13.75 s, 12.90 s und 12.24 s (PhOH), 7.92 dd (H-1), 7.72 t (H-2), 7.33 dd (H-3), 5.34 bs (H-7), 2.12 und 1.97 m (H-8a), 4.88 s (H-10), 2.68 m (H-13 a,b), 1.07 t (H-14), 0.25 s (MeSi)

7,10-Bis-0-(trimethylsilyl)-4-0-methyl-ß-rhodomycinon (Verbindung 2)

Eine Mischung von 1,13 g (2.13 mmol) Verbindung 1,20 ml Dichlormethan, 100 ml Aceton, 20 g (145 mmol) Kaliumcarbonat und 50 ml (800 mmol) Methyljodid wurden 12 Stunden bei Raumtemperatur geschüttelt. Die Reaktionsmischung wurde in Vakuum eingedampft, und der in Dichlormethan aufgenommene Rückstand wurde mit Eiswasser und anschließend mit 1 N Salzsäure ausgewaschen. Die organische Phase wurde über Natriumsulfat getrocknet. Der nach Abdampfen des Lösungsmittels erhaltene Rückstand wurde säulenchromatographisch an Kieselgel mit Dichlormethan als Eluierungsmittel gereinigt.

Ausbeute der Titelverbindung: 490 mg, Schmelzpunkt: 202° C (Zersetzung). Die Titelverbindung wurde mittels $^1$H-NMR-, MS- sowie Elementaranalyse charakterisiert.
$^1$H-NMR (400 MHz, $CDCl_3$,delta(ppm)): 13.92 s und 13.55 s (PhOH), 8.05 dd (H-1), 7.77 t (H-2), 7.38 dd (H-3), 5.35 bs (H-7), 2.15 dd (H-8a), 1.98 dd (H-8b), 4.87 s (H-10), 1.68 m (H-13a,b), 1.05 t (H-14), 4.08 s (OMe), 0.25 s (SiMe), 4.50 s (9-OH).

4-0-Methyl-ß-rhodomycinon (Verbindung 3)

Eine Lösung von 490 mg (0.9 mmol) Verbindung 2, gelöst in 50 ml Dichlormethan und 50 ml Methanol, wurde bei 0° C mit 10 ml 0.2 N Salzsäure versetzt und 15 min bei 0° C gerührt. Die Reaktionslösung wurde mit 500 ml Dichlormethan versetzt und mit 100 ml Eiswasser ausgewaschen. Die organische Phase wurde über Natriumsulfat getrocknet und in Vakuum eingedampft. Der Rückstand kristallisiert aus Dichlormethan/Ether.
Ausbeute der Titelverbindung: 346 mg (96 %) Schmelzpunkt: 141° C (Zersetzung), $(alpha)_D$ = +73° (c = 0.2 in Chloroform/Methanol 9:1)
$^1$H-NMR (400 MHz,$CDCl_3$,delta(ppm)): 13.38 s (6-OH), 13.86 s (11-OH), 8.02 dd (H-1, J(1,2) = 8.0 Hz, J-(1,3) = 1 Hz), 7.90 t (2-H), 7.40 dd (H-3, J(2,3) = 8.5 Hz, J(1,3) = 1 Hz), 5.25 bs (H-7), 2.15 ddd (H-8e, J-(8a,8e) = 14.0 Hz, J(7,8e) = 5 Hz, J(8e,10) = 2 Hz), 2.21 dd (H-8a, J(8a, 8e) = 14 Hz, J(7,8a) = 1 Hz), 4.88 d (H-10, J(10,10-OH) = 4 Hz), 1.77 m (H-13a, J(13a,13b) = 14.2 Hz, J(13a,14) = 7 Hz), 1.87 m (H-13b, J(13a,13b) = 14.2 Hz, J(13b,14) = 7 Hz), 1.12 t (H-14, J(13,14) = 7.0 Hz), 3.50 d (7-OH, J = 3.0 Hz), 3.47 s (9-OH), 2.66 d (10-OH, J = 4 Hz), 4.10 s (4-OMe) MS (160° C): m/z (%) = 400 (8,$M^+$), 382 (100), 364 (35), 328 (98), 326 (81), 325 (53), 311 (52), 310 (40), 297 (23)

Beispiel 2

Herstellung von 10-0-Acyl-4-0-alkyl-ß-rhodomycinonen

4-0-Methyl-7,9-0-phenylboronyl-ß-rhodomycinon (Verbindung 4)

800 mg (2 mmol) Verbindung 3, 4 g Molekularsieb 4 A, 732 mg Phenylboronsäure und 1.6 ml Eisessig wurden in 70 ml trockenem Toluol suspendiert. Dann wurde ein Drittel des Lösungsmittels abdestilliert und erneut 1.6 ml Eisessig zugegeben. Die Reaktionsmischung wurde ca. 50 Stunden bei 87° C gehalten, und der Reaktionsverlauf wurde dünnschichtchromatographisch (Toluol/ Methanol/Eisessig 10:1:1) kontrolliert. Die Reaktionsmischung wurde bei Raumtemperatur abfiltriert, und die organische Phase wurde mit verdünnter Natriumhydrogencarbonatlösung anschließend mit Wasser ausgewaschen und über Natriumsulfat getrocknet. Nach dem Abdampfen des Lösungsmittels wurde der Rückstand aus Chloroform/Petrolether ausgefällt. Das resultierende Rohprodukt wurde noch säulenchromatographisch über Kieselgel (Elutionsmittel: Toluol/Methanol/Eisessig 10:1:1) gereinigt.
Ausbeute: 640 mg, Schmelzpunkt: 230° C, $(alpha)_D$ = +440° C, (c = 0.2 in Chloroform)
$^1$H-NMR (300 MHz,$CDCl_3$,delta(ppm)): 7.79 d (H-1, J(1,2) = 8 Hz), 7.58 t (H-2, J(1,2) = J(2,3) = 8 Hz), 7.28 d (H-3, J(2,3) = 8), 5.61 t (H-7), 2.24 dd (H-8a, J(8,8) = 14 Hz, J(7,8) = 2.5 Hz), 2.10 dd (H-8b, J(8,8) = 14 Hz, J(8,10) = 2.3 Hz), 2.10 dd (H-8b, J(8,8) = 14 Hz, J(8,10) = 2.3 Hz), 2.07 m (H-13a, J(13,13) = 12 Hz, J(13,14) = 7.5 Hz), 1.78 m (H-13b, J(13,13) = 12 Hz, J(13,14) = 7.5 Hz), 1.17 t (H-14, J(13,14) = 7.5 Hz), 13.62 s und 13.23 s (PhOH), 8.16 m, 7.17 m und 7.21 m (PhB), 3.97 s (OMe).

4-0-Methyl-7,9-0-phenylboronyl-10-0-trifluoracetyl-ß-rhodomycinon (Verbindung 5)

300 mg (617 mmol) Verbindung 4 wurden in 20 ml trockenem Dichlormethan gelöst und bei 0° C mit 1.7 ml (20 Äquivalente) Trifluoressigsäure-Anhydrid gelöst mit 10 ml trockenem Dichlormethan versetzt. Unter Rühren wurde 0.1 ml Triethylamin zugegeben. Nach 18 Stunden (Dünnschichtplattenkontrolle mit Toluol/ Methanol/Eisessig 10:1:0.1) Rühren bei 4° C wurde der Reaktionsansatz in Vakuum eingedampft und zweimal mit Toluol nachdestilliert. Der Rückstand wurde mit Chloroform und Petrolether ausgefällt. Die Titelverbindung wurde ohne weitere Reinigung in der nächsten Stufe eingesetzt.
Ausbeute: 360 mg, Dünnschicht-Chromatogramm: Rf = 0.72 in Toluol/ Methanol/Eisessig 10:1:0.1

4-0-Methyl-10-0-trifluoracetyl-ß-rhodomycinon (Verbindung 6)

360 mg Rohprodukt der Verbindung 5 wurden in 10 ml trockenem Dichlormethan gelöst und mit 3 ml 2-Methylpentan-2,4-diol versetzt. Nach 18 Stunden Rühren bei Raumtemperatur wurde der Reaktionsansatz mit 10 ml Dichlormethan verdünnt und dreimal mit 0.01 N Salzsäure ausgewaschen. Die organische Phase wurde über Natriumsulfat getrocknet und in Vakuum eingedampft. Der Rückstand wurde säulenchromatographisch über Kieselgel mit Chloroform/Aceton/Eisessig/Wasser/Triethylamin 95:5:1:0.25:0.1 als Elutionsmittel gereinigt.
Ausbeute: 167 mg
$^1$H-NMR (400 MHz,$CDCl_3$,delta(ppm)): 7.94 dd (H-1, J(1,3) = 7.5 Hz, J(1,3) = 1.5 Hz), 7.78 t (H-2), 7.38 dd (H-3, J(2,3) = 8 Hz, J(1,3) = 1.5 Hz), 5.31 dd (H-7, J(7,8a) = 4.7 Hz, J(7,8b) = 1.5 Hz), 2.00 dd (H-8a, J-(8a,8b) = 15 Hz, J(7,8a) = 4.7 Hz), 2.37 ddd (H-8b, J(8a,8b) = 15 Hz, J(8b,10) = 1.5 Hz), 5.29 d (H-10, J-(8b,10) = 1.5 Hz), 1.71 m (H-13a, J(13,13) = 13.8 Hz, J(13,14) = 7.4 Hz), 1.53 m (H-13b, J(13,13) = 13.8 Hz, J(13,14) = 7.4 Hz), 1.09 t (H-14, J(13,14) = 7.4 Hz), 13.69 s und 13.03 s (PhOH), 4.08 s (MeO).

Beispiel 3

Herstellung von 7-0-Glycosyl-Derivaten

4-0-Methyl-7-0-(4′-0-p-nitrobenzoyl-3′-N-trifluoracetyl-L-daunosaminyl)-10-0-trifluoracetyl-ß-rhodomycinon (Verbindung 7)

1.67 g (3.36 mmol) Verbindung 6, 3.64 g (6.72 mmol) 1,4-Di-0-p-nitrobenzoyl-3-N-trifluoracetyl-alpha,ß-L-daunosamin und 5 g Molekularsieb 4A wurden in 300 ml eines Lösungsmittelgemisches aus Dichlormethan und Aceton 10:1 unter Feuchtigkeitsausschluß suspendiert. Der auf -50° C abgekühlte Reaktionsansatz

wurde mit 3 ml Trifluormethansulfonsäure-trimethylsilylester versetzt und 4 Stunden bei 44° C weitergerührt. Der Verlauf der Reaktion wurde dünnschichtchromatographisch kontrolliert (Laufmittel: Dichlormethan/Aceton 20:1, Titelverbindung: Rf = 0.74).
Die Reaktionsmischung wurde mit Triethylamin neutralisiert (pH 7) und abfiltriert. Nach dem Eindampfen der resultierenden Lösung wurde der Rückstand säulenchromatographisch gereinigt (Kieselgel, Elutionsmittel: Dichlormethan/Aceton 20:1).
Ausbeute: 2.18 g (75 % bezogen auf Aglycon) Schmelzpunkt: 128° C, $(alpha)_D$ = -124° (c = 0.1 in Chloroform), MS-FAB $M+H^+$ (m/z = 871), H,H-COSY (300 Mz).
$^1$H-NMR (300 MHz,$CDCl_3$,delta(ppm)): 8.05 dd (H-1, J(1,2) = 8 Hz, J(1,3) = 1 Hz), 7.83 t (H-2, J(1,2) = 8 Hz, J(2,3) = 8.5 Hz), 7.43 dd (H-3), 5.31 dd (H-7, J(7,8a) = 3.5 Hz, J(7,8b) = 1.5 Hz), 2.11 dd (H-8a, J(8,8) = 15 Hz, J(7,8a) = 3.5 Hz), 2.45 d (H-8b, J(8,8) = 15 Hz), 6.39 s (H-10), 1.78 m (H-13a, J(13,13) = 14 Hz, J(13a, 14) = 7.5 Hz), 1.52 m (H-13b, J(13,13) = 14 Hz, J(13b, 14) = 7.5 Hz), 1.10 t (H-14, J(13,14) = 7.5 Hz), 13.84 s und 13.22 s (PhOH), 4.10 s (MeO), 5.69 s (H-1′), 2.08 m (H-2′a), 2.11 m (H-2′e), 4.44 m (H-3′, J(3′,4′) = 2.5 Hz), 5.49 d (H-4′), 4.45 g (H-5′, J(5′6′) = 6.5 Hz), 1.27 d (H-6′, J(5′6′) = 6.5 Hz), 6.29 d (NH, J(NH,3′) = 7 Hz) 8.34 - 8.28 m (p-Nitrobenzoyl)

7-0-(4′-Desoxy-3′-N-trifluoracetyl-alpha-L-daunosaminyl)-4-0-methyl-10-0-trifluoracetyl-ß-rhodomycinon (Verbindung 8)

Ausgehend von 376 mg (1 mmol) 4-Desoxy-1-0-p-nitrobenzoyl,3-N-trifluoracetyl-alpha,ß-L-daunosamin und 496 mg (1 mmol) 4-0-Methyl-10-0-trifluoracetyl-ß-rhodomycinon (Verbindung 6) wurde die Titelverbindung in Analogie zur Herstellung der Verbindung 7 hergestellt.
Ausbeute der Titelverbindung: 557 mg (64 %).

4-0-Methyl-7-0-(4′-0-p-nitrobenzoyl-3′-N-trifluoracetyl-alpha-L-acosaminyl)-10-0-trifluoracetyl-ß-rhodomycinon (Verbindung 9)

260 mg (0.524 mmol) Verbindung 6 wurden in 20 ml trockenem Dichlormethan gelöst und mit 300 mg Molekularsieb und 294 mg (0.786 mmol) 1,5-Anhydro-4-0-p-nitrobenzoyl-2,3,6-tridesoxy-3-trifluoracetamido-L-arabino-hex-1-enit versetzt. 34.9 mg (0.157 mmol) Trifluormethansulfonsäuretrimethylsilylester wurden zu der auf -40° C abgekühlten Reaktionsmischung unter Schutzgasatmosphäre (Argon) zugegeben. Nach Aufwärmen auf -20° C wurde noch 4 Stunden gerührt und anschließend mit Triethylamin neutralisiert. Der Reaktionsansatz wurde abfiltriert und in Vakuum eingedampft. Das Rohprodukt wurde säulenchromatographisch gereinigt (Kieselgel, Elutionsmittel: Dichlormethan/Aceton 20:1).
Ausbeute: 392 mg (86 % bezogen auf Aglycon)

4-0-Methyl-7-0-(3′-N-trifluoracetyl-alpha-L-daunosaminyl)-ß-rhodomycinon (Verbindung 10)

200 mg (0.23 mmol) Verbindung 7 wurden in 5 ml einer Lösungsmittelmischung aus Methanol/Chloroform 3:2 gelöst, mit 5 ml 0.1 N wäßriger NaOH und anschließend mit Methanol bis Aufhebung der Phasen unter Rühren versetzt. Nach 30 min wurde mit 5 ml 0.1 N wäßriger HCl neutralisiert und in Vakuum eingedampft. Das Rohprodukt wurde säulenchromatographisch gereinigt (Kieselgel, Elutionsmittel: Chloroform/Aceton/Eisessig/ Wasser/Triethylamin 93:7:1:0.25:0.1).
Ausbeute der Titelverbindung: 118 mg (82 %).

7-0-(alpha-L-Daunosaminyl)-4-0-methyl-ß-rhodomycinon (Verbindung 11)

250 mg (0.287 mmol) Verbindung 7 wurden in 5 ml Methanol/Chloroform 3:2 gelöst, mit 5 ml 1 N wäßriger NaOH und anschließend mit Methanol bis Aufhebung der Phasen versetzt. Nach 4 Stunden Reaktionszeit wurde der Ansatz mit 5 ml 1 N wäßriger HCl neutralisiert und in Vakuum eingedampft. Das Rohprodukt wurde noch säulenchromatographisch gereinigt (Kieselgel: Chloroform/Aceton/Eisessig/Wasser/Triethylamin 90:9:1:0.25:0.1).
Ausbeute der Titelverbindung: 152 mg (72 %) MS-FAB $M+H^+$ (m/z = 530)

7-0-(4′-Desoxy-alpha-L-daunosaminyl)-4-0-methyl-ß-rhodomycinon (Verbindung 12)

Ausgehend von 500 mg (0.70 mmol) Verbindung 8 wurde die Titelverbindung nach dem Verfahren zur Herstellung von Verbindung 11 dargestellt.
Ausbeute: 232 mg (64 %)

7-0-(alpha-L-Acosaminyl)-4-0-methyl-ß-rhodomycinon (Verbindung 13)

Ausgehend von 250 mg (0.287 mmol) Verbindung 9 wurde die Titelverbindung nach dem Verfahren zur Herstellung von Verbindung 11 dargestellt. Ausbeute: 150 mg (70 %)

Beispiel 4

Modifizierung von 7-0-Glycosyl-4-0-methyl-ß-rhodomycinen an der 3′-Aminogruppe

4-0-Methyl-7-0-(alpha-L-rhodosaminyl)-ß-rhodomycinon (Verbindung 14)

200 mg (0.378 mmol) Verbindung 11 wurden in 20 ml Methanol gelöst und mit 0.62 ml 37 %iger Formaldehyd-Lösung (20 äq) unter Rühren bei Raumtemperatur versetzt. Nach der Zugabe von 142 mg (2.27 mmol) Natriumcyanoborhydrid wurde der Reaktionsansatz noch 3 h gerührt und nach dünnschichtchromatographischer Kontrolle (Laufmittel: Chloroform/Methanol/Eisessig/Wasser/Triethylamin 6:2:1: 0.6:0.05) in Vakuum eingedampft. Das resultierende Rohprodukt wurde an Kieselgel säulenchromatographisch gereinigt.
Ausbeute: 155 mg (74 %)

4-0-Methyl-7-0-(3′-(4″-morpholinyl)-2′3′6′-tridesoxy-alpha-L-lyxohexopyranosyl)-ß-rhodomycinon (Verbindung 15)

200 mg (0.378 mmol) Verbindung 11 wurden in 20 ml Methanol/Chloroform 10:1 gelöst und mit 77.2 mg (0.756 mmol) 2,2′-Oxy-bis-acetyldehyd, das frisch aus 1,4-Anhydroerythritol durch Perjodatoxidation zubereitet wurde, versetzt. Nach der Zugabe von 24 mg (0.378 mmol) Natriumcyanoborhydrid wurde der Reaktionsansatz 2 Stunden bei Raumtemperatur gerührt und dann in Vakuum eingedampft. Der Rückstand wurde säulenchromatographisch gereinigt (Kieselgel, Eluationsmittel: Chloroform/Aceton/Eisessig/Wasser/Triethylamin 90:9:1:0.25:0.1).
Ausbeute: 164 mg (73 %)

7-0-(3′-N-Cyanomethyl-alpha-L-daunosaminyl)-4-0-methyl-ß-rhodomycinon (Verbindung 16)

200 mg (0.378 mmol) Verbindung 11 wurden in 20 ml DMF gelöst und mit 115 mg (1.134 mmol = 3 äq) Triethylamin und 631 mg (3.78 mmol) Jodacetonitril versetzt. Nach 5 Stunden Rühren bei Raumtemperatur wurden weitere 230 mg (2.268 mmol) Triethylamin zugegeben. Der Reaktionsansatz wurde noch 14 Stunden gerührt und anschließend eingedampft. Die säulenchromatographische Reinigung erfolgte an Kieselgel mit dem Eluationmittel: Chloroform/Aceton/Eisessig/Wasser/Triethylamin 90:9:1:0.4:0.1.
Ausbeute: 137 mg (64 %)

Beispiel 5

Ermittlung der zytostatischen Aktivität

Die Bestimmung der zytostatischen Wirksamkeit der hier beschriebenen Verbindungen erfolgte an

L1210-Leukämiezellen der Maus. Im einzelnen wurden folgende Testsysteme verwendet:

a) Koloniebildung von L1210-Leukämiezellen in soft agar

Diese Methode diente zum Nachweis eines Einflusses der Testsubstanzen auf das Wachstumsverhalten der Zellen über mehrere Generationen (bei einer Zellzykluszeit von 10 - 12 Stunden werden in der Testzeit von 7 Tagen etwa 14 aufeinanderfolgende Generationen beobachtet). Zytostatisch wirksame Substanzen bewirkten in diesem Test eine Reduktion der zu beobachtenden Koloniezahl gegenüber einer unbehandelten Kontrolle. Im einzelnen wurde der Test wie folgt durchgeführt:

500 Leukämiezellen pro Platte wurden mit unterschiedlichen Konzentrationen von Testsubstanz 1 Stunde bei 37° C inkubiert.

Anschließend wurden die Zellen zweimal mit McCoy5A-Medium gewaschen und schließlich in Petrischalen nach Zusatz von 0.3 % Agar ausgegossen. Kontrollen wurden lediglich mit frischem Medium inkubiert. Anstelle der einstündigen Inkubation wurden in manchen Fällen unterschiedliche Konzentrationen von Testsubstanz der oberen Agarschicht zugemischt, um so eine kontinuierliche Expo sition der Zellen über die gesamte Inkubationszeit zu erreichen. Nach Erstarren des Agars wurden die Platten im Brutschrank 7 Tage bei 37° C inkubiert ( 5 Vol-% $CO_2$, 95 % der relativen Luftfeuchtigkeit). Anschließend wurde die Anzahl der entstandenen Kolonien mit einem Durchmesser von mehr als 60 $\mu$ gezählt. Die Ergebnisse wurden angegeben als Koloniezahl in behandelten Agarplatten, in Prozent der unbehandelten Kontrolle. Aus der so erhaltenen Dosiswirkungskurve wurde die $IC_{50}$ als Maß für die Wirksamkeit der Substanz ermittelt. Die Ergebnisse für die hier beschriebenen Verbindungen im Vergleich zu Adriamycin wurden ermittelt.

b) Ermittlung der akuten Toxizität

Zur Ermittlung der akuten Toxizität wurden NMRI-Mäusen am Tag 0 unterschiedliche Dosen der Testsubstanz gelöst in 0.5 ml 5 %iger Glucose-Lösung, intraperitoneal injiziert. Kontrollgruppen erhielten lediglich 0.5 ml 5 %ige Glucose-Lösung. Pro Konzentration der Testsubstanz wurden 5 Mäuse verwendet. Am Tag 14 wurde die Zahl der überlebenden Mäuse ermittelt und daraus nach der Lichtfield Wilcoxon Methode die LD5, LD50 und LD95 ermittelt. Die Toxizität LD50 (mg/kg) der hier beschriebenen Verbindungen im Vergleich zu Adriamycin wurde ermittelt.

c) In vivo Wirksamkeit der Rhodomycine gegen L1210 Leukämie der Maus

Methodik:

Ascitesflüssigkeit wurde unter sterilen Bedingungen DBA2 Mäusen (weiblich, 18 - 20 g) 7 Tage nach Implantation entnommen. Der Ascites wurde dreimal mit PBS gewaschen, gezählt und auf eine Zellzahl von $10^6$ in 0.2 ml PBS eingestellt.

$10^6$ Zellen, suspendiert in 0.2 ml PBS, wurden anschliessend DBF1 Mäusen (weiblich, 18 - 20 g) intraperitoneal injiziert. 6 Tiere pro Gruppe wurden für jede Substanzkonzentration bzw. als Kontrolle eingesetzt.

Ermittlung der antitumoralen Wirksamkeit:

a) Die Tiere wurden am Tag 1 und 5 nach Injektion der Testsubstanz gewogen. Gewichtsverlust von mehr als 20 % am Tag 5 wurde als Indikator einer toxischen Substanzwirkung angesehen.

b) Am Ende des Experimentes (Tod aller Tiere oder überlebende Tiere am Tag 60) wurde die mittlere Überlebenszeit der Tiere in den jeweiligen Gruppen bestimmt, sofern am Tag 5 des Experimentes mindestens 65 % der Tiere noch gelebt hatten. Die mittlere Überlebenszeit wurde ausschließlich für im Verlaufe des Experimentes sterbende Tiere bestimmt. Langzeitüberlebende (LTS) wurden bei dieser Berechnung nicht berücksichtigt und gesondert aufgeführt.

Aus der mittleren Überlebenszeit ($MST_t$) der behandelten Gruppen sowie der Kontrollgruppen ($MST_c$) wurde die antitumorale Wirksamkeit (T/C) für die jeweilige Substanzkonzentration in Prozent der unbehandelten

Kontrolle entsprechend der folgenden Formel bestimmt:

$$T/C\ \% = \frac{MST_T}{MST_C} \times 100$$

T/C-Werte größer als 125 % wurden als Indikator einer signifikanten antitumoralen Wirksamkeit der Testsubstanz angesehen. Die Dosis mit dem größten antitumoralen Effekt (optimale Dosierung) sowie jeweils eine Dosisstufe oberhalb und unterhalb dieser Dosis wurden ermittelt. Tiere, die am Tag 60 des Experimentes noch lebten, wurden als Longterm-Survivors getrennt aufgeführt.

**Ansprüche**

1. Neue zytostatisch wirksame Anthracyclin-Derivate, die der nachfolgenden allgemeinen Formel I

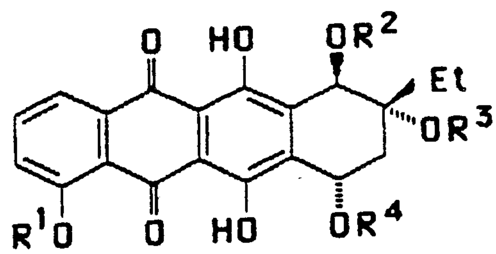

I

entsprechen, worin die Reste folgende Bedeutung haben:
$R^1$ ist eine $C_{1-4}$-Alkylgruppe,
$R^2$ ist ein Wasserstoffatom, eine Tri-$C_{1-4}$-alkylsilyl-Schutzgruppe, eine Acetyl-, Monohalogen-, Dihalogen- oder Trihalogen-acetylgruppe mit Halogen gleich Fluor oder Chlor, eine Benzoyl- oder p-Nitrobenzoylgruppe,
$R^3$ ist ein Wasserstoffatom,
$R^3$ und $R^4$ sind zusammen ein Phenylboronsäurerest PhB =,
$R^4$ ist ein Wasserstoffatom, eine Tri-$C_{1-4}$-alkylsilyl-Schutzgruppe oder ein Glycosyl-Rest der Formel II,

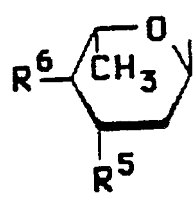

II

in der
$R^5$ eine Hydroxygruppe, eine Acetyloxygruppe, eine Aminogruppe, eine Trifluoracetamidogruppe, eine Di-$C_{1-4}$-alkylaminogruppe, eine 4-Morpholinylgruppe oder eine Cyanomethylaminogruppe und
$R^6$ ein Wasserstoffatom, eine Hydroxygruppe, eine Acetyloxy-, Trifluoracetyloxy- oder p-Nitrobenzoyloxy-Gruppe darstellen.

2. Verbindungen der Formel I, worin
$R^1$ = $CH_3$-,
$R^2$ = $(Me)_3Si$-, $F_3CCO$-,
$R^3$ = H,
$R^3$ und $R^4$ zusammen Ph-B =,
$R^4$ = H, $Me_3Si$- oder ein Glycosyl-Rest der Formel II mit

$R^5$ = OH, $CH_3COO$-, $NH_2$-, $F_3CCONH$-, $Me_2N$-, 4-Morpholinyl oder $NCCH_2NH$ und
$R^6$ = H, OH, $CH_3COO$-, $F_3CCOO$-, $pNO_2$-PhCOO- bedeuten.

3. Verfahren zur Herstellung von 4-0-Alkyl-ß-rhodomycinon-Zwischenverbindungen der Formel I, **dadurch gekennzeichnet,** daß man ß-Rhodomycinon der Formel I, worin die Reste $R^1$, $R^2$, $R^3$ und $R^4$ ein Wasserstoffatom darstellen, mit Tri-$C_{1-4}$-alkylsilylchlorid in Gegenwart einer Base wie Pyridin und eines organischen Lösungsmittels wie Dichlormethan bei einer Temperatur zwischen -20° C und +25° C zum 7,10-Bis-0-tri-$C_{1-4}$-alkylsilyl-ß-rhodomycinon umsetzt, anschließend die entstandene Verbindung mit $C_{1-4}$-Alkylbromid oder -jodid in Gegenwart eines Alkalicarbonats zu einem 4-0-$C_{1-4}$-Alkyl-7,10-bis-0-(tri-$C_{1-4}$-alkylsilyl)-ß-rhodomycinon alkyliert und dann die Trialkylsilyl-Schutzgruppen säurehydrolytisch abspaltet, wobei eine 4-0-$C_{1-4}$-Alkyl-ß-rhodomycinon-Verbindung der Formel III entsteht,

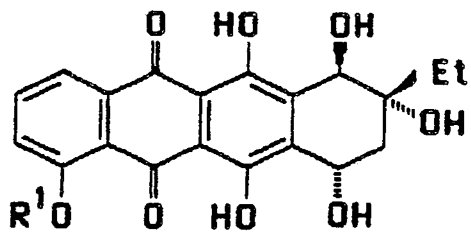
III,

worin $R^1$ eine $C_{1-4}$-Alkylgruppe darstellt.

4. Verfahren zur Herstellung von 10-0-Acyl-4-0-alkyl-ß-rhodomycinon-Zwischenverbindungen der Formel I, **dadurch gekennzeichnet,** daß man eine Verbindung der Formel III in an sich bekannter Weise mit Phenylboronsäure in Gegenwart einer Säure wie p-Toluolsulfonsäure oder Essigsäure und eines wasserfreien organischen Lösungsmittels wie Toluol selektiv an der 7- und 9-Hydroxygruppe verestert, anschließend die Verbindung mit einem Anhydrid oder Chlorid der Trifluoressig-, Essig- Benzoe- oder p-Nitrobenzoe-Säure in Präsenz einer Base wie Triethylamin oder Pyridin an der 10-Hydroxygruppe acyliert und dann die Phenylboronyl-Schutzgruppe säurehydrolytisch oder mittels eines Diols wie 2-Methyl-pentan-2,4-diol abspaltet, wobei eine Verbindung der Formel IV entsteht,

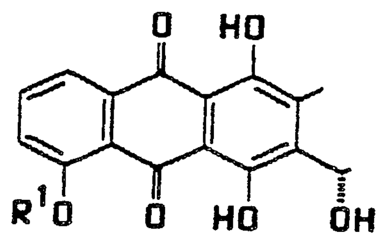
IV,

in der
$R^1$ eine $C_{1-4}$-Alkylgruppe und
$R^2$ eine Acylschutzgruppe, die sich von der Essig-, Trifluoressig-, Benzoe- oder p-Nitrobenzoesäure ableitet, bedeuten.

5. Verfahren zur Herstellung von 10-0-Alkyl-7-0-glycosyl-ß-rhodomycinon der Formel I, **dadurch gekennzeichnet,** daß man die Verbindung der Formel IV in an sich bekannter Weise mit einem funktionalisierten Desoxyzucker der Formel V oder VI,

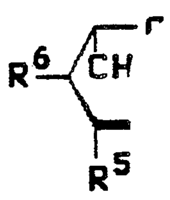
V

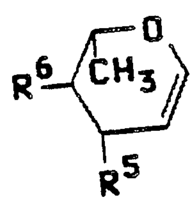
VI

worin die Reste
$R^5$ eine Acetyloxy- oder Trifluoracetamidogruppe,

$R^6$ ein Wasserstoffatom, eine Acetyloxy-, Trifluoracetyloxy- oder p-Nitrobenzoyloxygruppe und
Y eine Acetyloxy-, p-Nitrobenzoyloxygruppe oder ein Chlorid darstellen,
in Gegenwart eines Katalysators wie eines Trifluormethansulfonsäure-tri-$C_{1-4}$-alkylsilylesters oder dem Silbersalz der Trifluormethansulfonsäure zu einem 7-0-Glycosyl-ß-rhodomycinon-Derivat umsetzt und anschließend die Acylschutzgruppe alkalihydrolytisch abspaltet, wobei eine Verbindung der Formel VII entsteht,

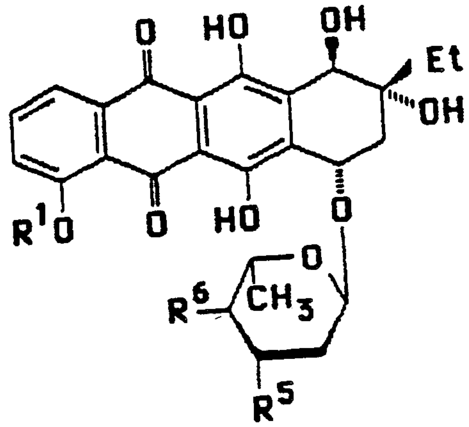

VII,

worin die Reste
$R^1$ eine $C_{1-4}$-Alkylgruppe,
$R^5$ eine Hydroxy- oder Aminogruppe und
$R^6$ ein Wasserstoffatom oder eine Hydroxygruppe darstellen.

6. Verfahren zur Herstellung einer modifizierten 7-0-Glycosyl-Verbindung der Formel I, **dadurch gekennzeichnet,** daß man eine einen Aminozucker enthaltende Verbindung der Formel VII in an sich bekannter Weise gegebenenfalls unter den Bedingungen der Alkylierung mit einem Halogenessigsäurenitril, oder der reduktiven Alkylierung mit $C_{1-4}$-Aldehyd oder Diglycolaldehyd in Gegenwart eines Alkalicyanoborhydrides zu einer weiteren Verbindung der Formel VII umsetzt, in der die Reste
$R^1$ eine $C_{1-4}$-Alkylgruppe,
$R^5$ eine Cyanomethylaminogruppe, eine Di-$C_{1-4}$-alkylaminogruppe oder eine 4-Morpholinylgruppe und
$R^6$ ein Wasserstoffatom oder eine Hydroxygruppe darstellen.

7. Verwendung einer Verbindung nach Anspruch 1 in einem Arzneimittel.

Patentansprüche für folgende Vertragsstaaten: ES, GR

1. Verfahren zur Herstellung von 4-0-Alkyl-ß-rhodomycinon-Zwischenverbindungen der Formel I,

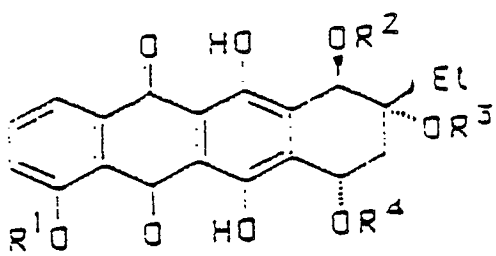

I

worin die Reste folgende Bedeutung haben:
$R^1$ ist eine $C_{1-4}$-Alkylgruppe,
$R^2$ ist ein Wasserstoffatom, eine Tri-$C_{1-4}$-alkylsilyl-Schutzgruppe, eine Acetyl-, Monohalogen-, Dihalogen- oder Trihalogen-acetylgruppe mit Halogen gleich Fluor oder Chlor, eine Benzoyl- oder p-Nitrobenzoylgruppe,
$R^3$ ist ein Wasserstoffatom,
$R^3$ und $R^4$ sind zusammen ein Phenylboronsäurerest PhB=,
$R^4$ ist ein Wasserstoffatom, eine Tri-$C_{1-4}$-alkylsilyl-Schutzgruppe oder ein Glycosyl-Rest der Formel II,

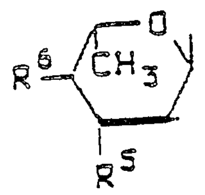

II

in der
$R^5$ eine Hydroxygruppe, eine Acetyloxygruppe, eine Aminogruppe, eine Trifluoracetamidogruppe, eine Di-$C_{1-4}$-alkylaminogruppe, eine 4-Morpholinylgruppe oder eine Cyanomethylaminogruppe und
$R^6$ ein Wasserstoffatom, eine Hydroxygruppe, eine Acetyloxy-, Trifluoracetyloxy- oder p-Nitrobenzoyloxy-Gruppe darstellen,
**dadurch gekennzeichnet**, daß man ß-Rhodomycinon der Formel I, worin die Reste $R^1$, $R^2$, $R^3$ und $R^4$ ein Wasserstoffatom darstellen, mit Tri-$C_{1-4}$-alkylsilylchlorid in Gegenwart einer Base wie Pyridin und eines organischen Lösungsmittels wie Dichlormethan bei einer Temperatur zwischen -20° C und +25° C zum 7,10-Bis-0-tri-$C_{1-4}$-alkylsilyl-ß-rhodomycinon umsetzt, anschließend die entstandene Verbindung mit $C_{1-4}$-Alkylbromid oder -jodid in Gegenwart eines Alkalicarbonats zu einem 4-0-$C_{1-4}$-Alkyl-7,10-bis-0-(tri-$C_{1-4}$-alkylsilyl)-ß-rhodomycinon alkyliert und dann die Trialkylsilyl-Schutzgruppen säurehydrolytisch abspaltet, wobei eine 4-0-$C_{1-4}$-Alkyl-ß-rhodomycinon-Verbindung der Formel III entsteht,

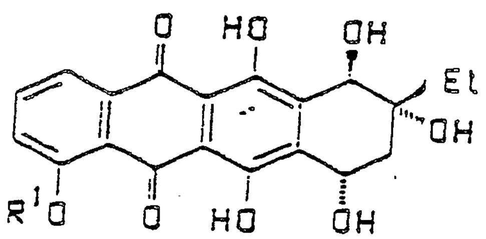

III,

worin $R^1$ eine $C_{1-4}$-Alkylgruppe darstellt.

2. Verfahren zur Herstellung von 10-0-Acyl-4-0-alkyl-ß-rhodomycinon-Zwischenverbindungen der Formel I, **dadurch gekennzeichnet,** daß man eine Verbindung der Formel III in an sich bekannter Weise mit Phenylboronsäure in Gegenwart einer Säure wie p-Toluolsulfonsäure oder Essigsäure und eines wasserfreien organischen Lösungsmittels wie Toluol selektiv an der 7- und 9-Hydroxygruppe verestert, anschließend die Verbindung mit einem Anhydrid oder Chlorid der Trifluoressig-, Essig- Benzoe- oder p-Nitrobenzoe-Säure in Präsenz einer Base wie Triethylamin oder Pyridin an der 10-Hydroxygruppe acyliert und dann die Phenylboronyl-Schutzgruppe säurehydrolytisch oder mittels eines Diols wie 2-Methylpentan-2,4-diol abspaltet, wobei eine Verbindung der Formel IV entsteht,

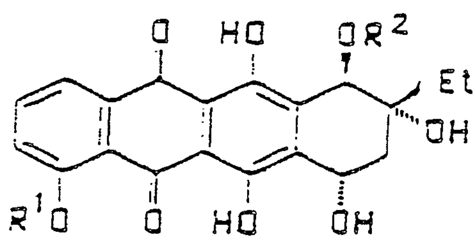

IV,

in der
$R^1$ eine $C_{1-4}$-Alkylgruppe und
$R^2$ eine Acylschutzgruppe, die sich von der Essig-, Trifluoressig-, Benzoe- oder p-Nitrobenzoesäure ableitet, bedeuten.

3. Verfahren zur Herstellung von 10-0-Alkyl-7-0-glycosyl-ß-rhodomycinon der Formel I, **dadurch gekennzeichnet,** daß man die Verbindung der Formel IV in an sich bekannter Weise mit einem funktionalisierten Desoxyzucker der Formel V oder VI,

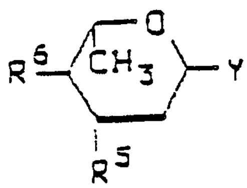 V 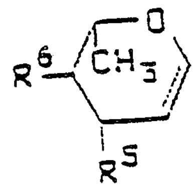 VI

worin die Reste
$R^5$ eine Acetyloxy- oder Trifluoracetamidogruppe,
$R^6$ ein Wasserstoffatom, eine Acetyloxy-, Trifluoracetyloxy- oder p-Nitrobenzoyloxygruppe und
Y eine Acetyloxy-, p-Nitrobenzoyloxygruppe oder ein Chlorid darstellen,
in Gegenwart eines Katalysators wie eines Trifluormethansulfonsäure-tri-$C_{1-4}$-alkylsilylesters oder dem Silbersalz der Trifluormethansulfonsäure zu einem 7-0-Glycosyl-ß-rhodomycinon-Derivat umsetzt und anschließend die Acylschutzgruppe alkalihydrolytisch abspaltet, wobei eine Verbindung der Formel VII entsteht,

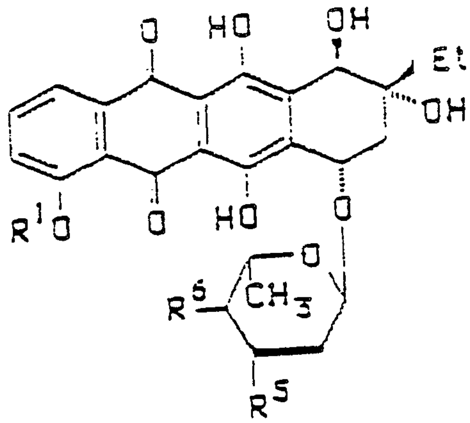 VII,

worin die Reste
$R^1$ eine $C_{1-4}$-Alkylgruppe,
$R^5$ eine Hydroxy- oder Aminogruppe und
$R^6$ ein Wasserstoffatom oder eine Hydroxygruppe darstellen.

4. Verfahren zur Herstellung einer modifizierten 7-0-Glycosyl-Verbindung der Formel I, **dadurch gekennzeichnet,** daß man eine einen Aminozucker enthaltende Verbindung der Formel VII in an sich bekannter Weise gegebenenfalls unter den Bedingungen der Alkylierung mit einem Halogenessigsäurenitril, oder der reduktiven Alkylierung mit $C_{1-4}$-Aldehyd oder Diglycolaldehyd in Gegenwart eines Alkalicyanoborhydrides zu einer weiteren Verbindung der Formel VII umsetzt, in der die Reste
$R^1$ eine $C_{1-4}$-Alkylgruppe,
$R^5$ eine Cyanomethylaminogruppe, eine Di-$C_{1-4}$-alkylaminogruppe oder eine 4-Morpholinylgruppe und
$R^6$ ein Wasserstoffatom oder eine Hydroxygruppe darstellen.